# EUROPEAN PATENT APPLICATION

(11) **EP 4 527 847 A1**
(43) Date of publication of application: **26.03.2025**
(21) Application number: 23806957.9
(22) Date of filing: 17.05.2023
(51) Int. Cl.: C07K 5/062, A61K 38/06, A61P 31/14

(54) **CRYSTAL FORMS OF HS378 AND PREPARATION METHOD THEREFOR**

(30) Priority: 19.05.2022 CN 202210552617
(71) Applicant: Zhejiang Hisun Pharmaceutical Co., Ltd., Taizhou, Zhejiang 318000 (CN)
(72) Inventor: LI, Na, Taizhou, Zhejiang 318000 (CN); SHI, Zhenjuan, Taizhou, Zhejiang 318000 (CN); CHEN, Lianwei, Taizhou, Zhejiang 318000 (CN); ZHAO, Meiyu, Taizhou, Zhejiang 318000 (CN); YANG, Zhiqing, Taizhou, Zhejiang 318000 (CN); WU, Zhongwei, Taizhou, Zhejiang 318000 (CN)
(74) Representative: Dehns
(86) International application number: PCT/CN2023/094661
(87) International publication number: WO 2023/222006

(57) **Abstract**

The present invention relates to crystal forms A and B of a compound (HS378) of formula (I) and a preparation method therefor. The crystal forms A and B have a good physiochemical stability, a regular crystal habit, a good particle size uniformity, and a good fluidity; the preparation method for the crystal forms A and B is simple; moreover, a good impurity removal effect, easiness in filtration and a high yield are achieved, and samples with few impurities and a high purity can be obtained; and the purification problem of difficulties during impurity removal can be fully solved.

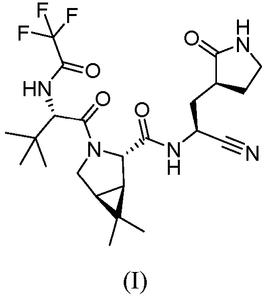

## Description

### TECHNICAL FIELD

The present disclosure relates to the field of chemical pharmaceutical. More specifically, the present disclosure relates to crystal forms A and B of (1R, 2S, 5S)-N-{(1S)-1-cyano-2-[(3S)-2-oxopyrrolidin-3-yl]-ethyl}-6,6-dimethyl-3-[3-methyl-N-(trifluoro acetyl)-L-valyl]-3-azabicyclo[3.1.0]hexane-2-carboxamide (HS378), a method for preparing the crystal forms, and medical uses thereof.

### BACKGROUND

The global coronavirus disease broke out in 2019 and new coronavirus has become a global epidemic. The novel coronavirus is a highly contagious ribonucleic acid coronavirus that causes life-threatening viral pneumonia in the most severe cases. Like vaccines, antiviral therapy is an important component of medical countermeasures against new coronavirus. HS378 is an orally bioavailable SARSCoV-2 major protease inhibitor with in vitro coronavirus antiviral activity, excellent off-target selectivity and in vivo safety. Oral activity was demonstrated in a mouse-adapted model of novel coronavirus, and oral plasma concentration exceeding in vitro antiviral cell efficacy were achieved in a Phase I clinical trial in healthy human participants.

The chemical name of the compound of formula (I) is (1R, 2S, 5S)-N-{(1S)-1-cyano-2-[(3S)-2-oxopyrrolidin-3-yl]-ethyl}-6,6-dimethyl-3-[3-methyl-N-(trifluoro acetyl)-L-valyl]-3-azabicyclo[3.1.0]hexane-2-carboxamide (named HS378 in this application), and its structural formula is shown below:

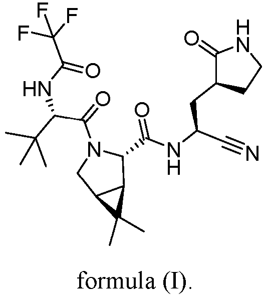

At present, Pfizer's patent WO2021250648A discloses crystal form 1, crystal form 2, crystal form 4, and crystal form 5 of HS378. Crystal form 2 is a methyl tert-butyl ether solvate, and crystal form 5 is amorphous. Crystal form 1, crystal form 4 and crystal form 5 are all prepared by using crystal form 2 as a substrate, and there are two impurities that are difficult to remove during the process, and the relative retention times of these two impurities are RRT=0.96 and RRT=1.04. The crystallization process of crystal forms 1, 2, 4, and 5 cannot effectively remove the two impurities. Therefore, it is necessary to develop new crystal forms and new processes.

### SUMMARY

Therefore, in view of the problems existing in the above-mentioned prior art, the present disclosure provides crystal form A and crystal form B of HS378, and their crystallization methods have better impurity removal effect.

The X-ray powder diffraction pattern of the crystal form A of HS378 described in the present disclosure (hereinafter referred to crystal form A) has characteristic peaks at the following diffraction angles 2θ: 6.2±0.2°, 6.9±0.2°, 7.9±0.2°, 10.5±0.2°, 11.5±0.2°, 11.9±0.2°, 12.5±0.2°, 13.9±0.2°, 14.3±0.2°, 15.8±0.2°, 18.7±0.2°, 19.4±0.2°, 20.7±0.2° and 23.3±0.2°.

Furthermore, the X-ray powder diffraction pattern of the crystal form A of the present disclosure has 2θ values, interplanar spacing d values and relative intensity data as shown in Table 1 below:

**Table 1**

| Peak number | 2θ(°) | d(A) | Relative intensity (%) |
|---|---|---|---|
| 1 | 6.2 | 14.2 | 11.5 |
| 2 | 6.9 | 12.8 | 25.4 |
| 3 | 7.9 | 11.2 | 5.6 |
| 4 | 10.5 | 8.4 | 9.0 |
| 5 | 11.5 | 7.7 | 38.7 |
| 6 | 11.9 | 7.4 | 61.5 |
| 7 | 12.5 | 7.1 | 37.5 |
| 8 | 13.9 | 6.4 | 26.8 |
| 9 | 14.3 | 6.2 | 86.1 |
| 10 | 15.8 | 5.6 | 59.6 |
| 11 | 18.7 | 4.7 | 100 |
| 12 | 19.4 | 4.6 | 36.9 |
| 13 | 20.7 | 4.3 | 35.4 |
| 14 | 23.3 | 3.8 | 37.6 |

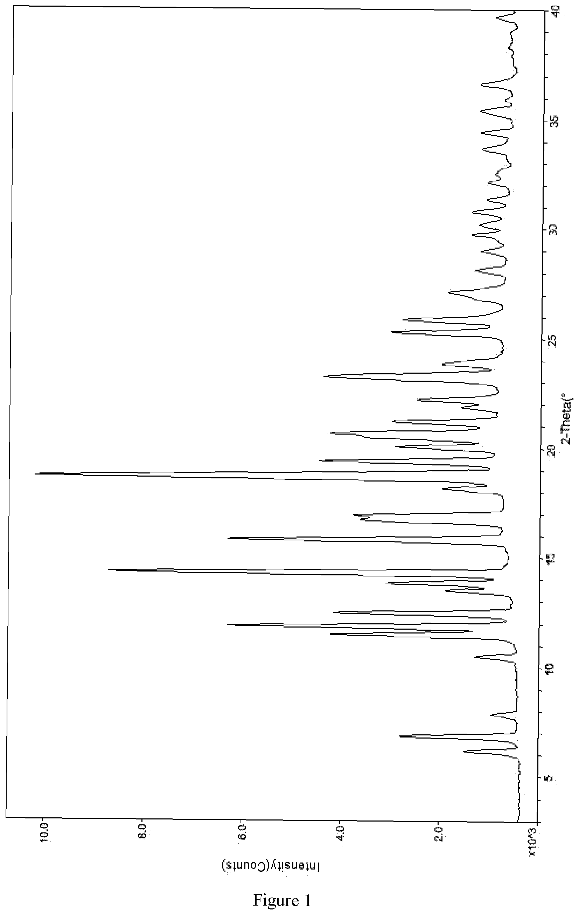

Without limitation, the X-ray powder diffraction pattern of the crystal form A of the present disclosure is basically as shown in Figure 1.

The differential scanning calorimetry (DSC) pattern of the crystal form A of the present disclosure has an endothermic peak with a Peak value of 105.2°C.

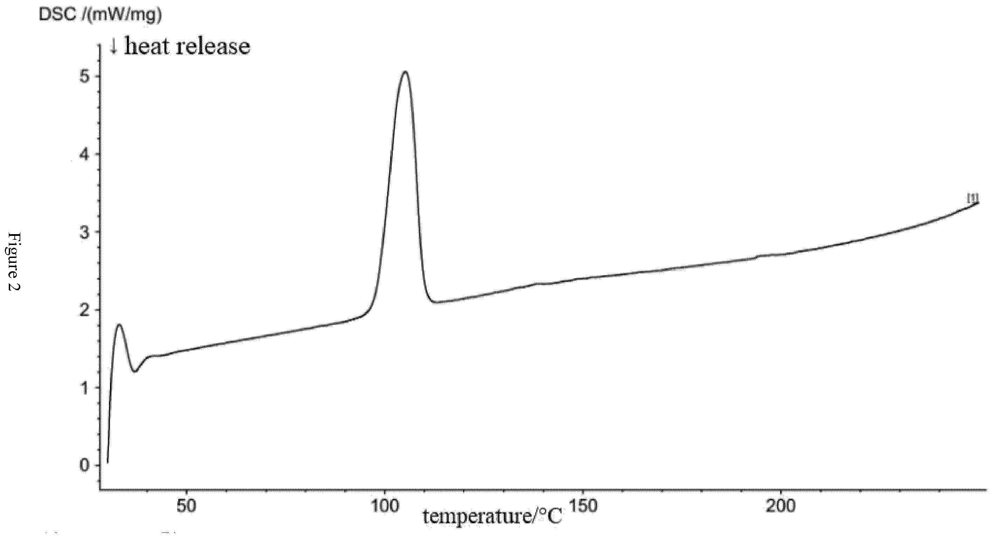

Without limitation, the crystal form A of the present disclosure has a DSC pattern as shown in Figure 2.

The thermogravimetric analyzer (TGA) pattern of the crystal form A of the present disclosure has a weight loss.

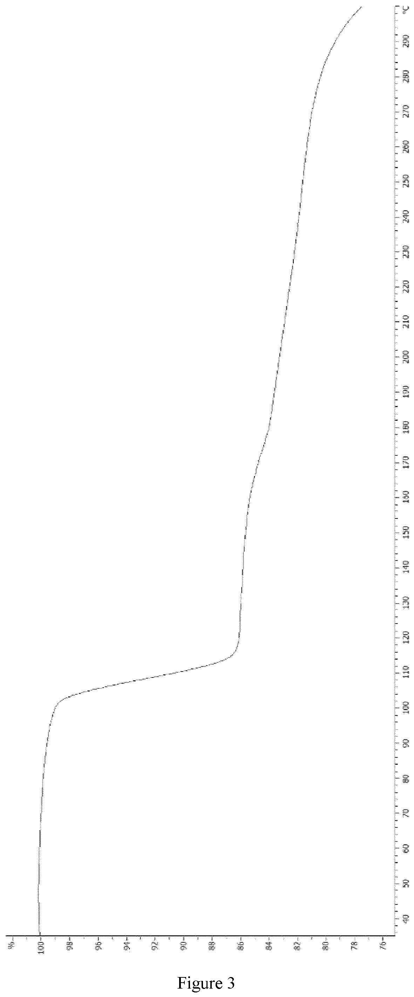

Without limitation, the crystal form A of the present disclosure has a TGA pattern as shown in Figure 3.

Combining the data of DSC and TGA, it is shown that the crystal form A of the present disclosure is an isobutyl acetate solvate.

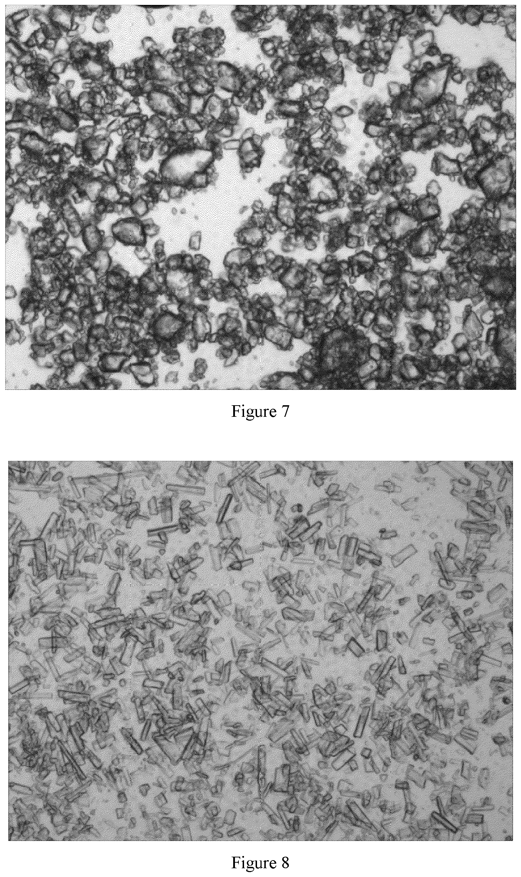

Without limitation, the crystal form A of the present disclosure has a microscope photograph as shown in Figure 7, the crystal has a blocky crystal habit, close to spherical, and the friction between particles is small and the fluidity is good.

Another object of the present disclosure is to provide a method for preparing the crystal form A, comprising the following steps:
(1) mixing a crude product of compound of formula (I) with a mixed solvent of alcohol and isobutyl acetate;
(2) stirring and crystallizing for 1-24 h;
(3) filtering to obtain the crystal form A.

Preferably, wherein, in the above step (1), a mass volume ratio of the crude product of compound of formula (I) to the mixed solvent is 1:5-40, and its unit is g/ml; a volume ratio (ml/ml) of the alcohol to isobutyl acetate is 1:7-1:100; the alcohol is C1-C2 alcohol; and a temperature for the mixing is 25-80°C.

Preferably, a temperature for stirring and crystallizing in step (2) is 5-25°C.

The X-ray powder diffraction pattern of the crystal form B of compound of formula (I) of the present disclosure (hereinafter referred to crystal form B) has characteristic peaks at the following diffraction angles 2θ: 6.7±0.2°, 7.4±0.2°, 9.3±0.2°, 10.0±0.2°, 10.9±0.2°, 11.2±0.2°, 15.8±0.2°, 16.4±0.2°, 17.4±0.2°, 18.4±0.2°, 19.4±0.2°, 20.3±0.2° and 20.8±0.2°.

Furthermore, the X-ray powder diffraction pattern of the crystal form B of the present disclosure has 2θ values, interplanar spacing d values and relative intensity data as shown in Table 2 below:

**Table 2**

| Peak number | 2θ(°) | d(A) | Relative intensity (%) |
|---|---|---|---|
| 1 | 6.7 | 13.2 | 13.8 |
| 2 | 7.4 | 12.0 | 7.1 |
| 3 | 9.3 | 9.5 | 32.7 |
| 4 | 10.0 | 8.8 | 57.1 |
| 5 | 10.9 | 8.1 | 33.1 |
| 6 | 11.2 | 7.9 | 44.3 |
| 7 | 15.8 | 5.6 | 72.7 |
| 8 | 16.4 | 5.4 | 59.8 |
| 9 | 17.4 | 5.1 | 66.9 |
| 10 | 18.4 | 4.8 | 100 |
| 11 | 19.4 | 4.6 | 71 |
| 12 | 20.3 | 4.4 | 55.9 |
| 13 | 20.8 | 4.3 | 40.4 |

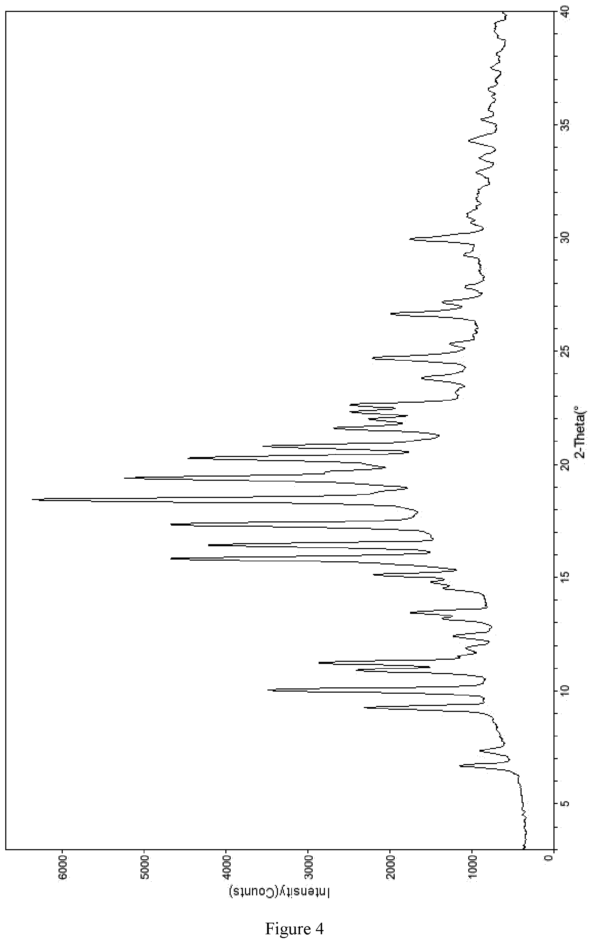

Without limitation, the X-ray powder diffraction pattern of the crystal form B of the present disclosure is basically as shown in Figure 4.

The differential scanning calorimetry (DSC) pattern of the crystal form B of the present disclosure has an endothermic peak with a Peak value of 94.4°C.

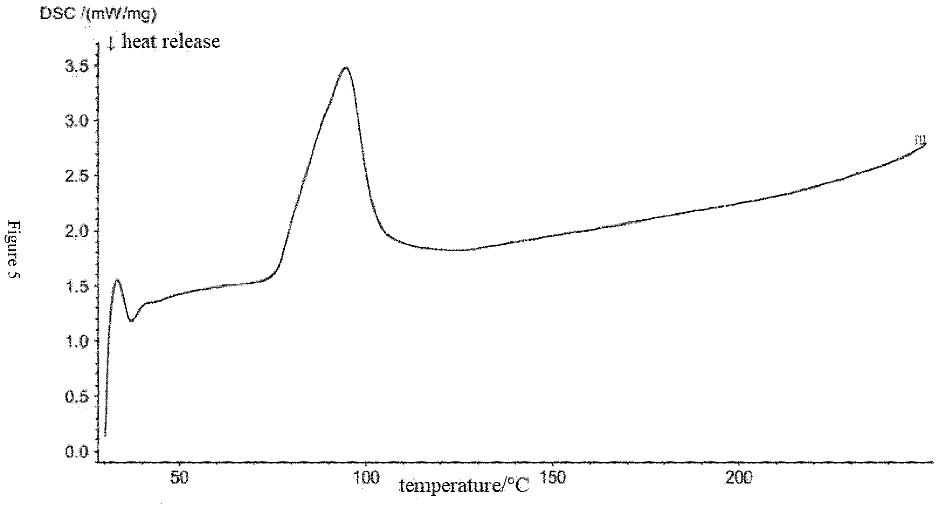

Without limitation, the crystal form B of the present disclosure has a DSC pattern as shown in Figure 5.

The thermogravimetric analyzer (TGA) pattern of the crystal form B of the present disclosure has a weight loss.

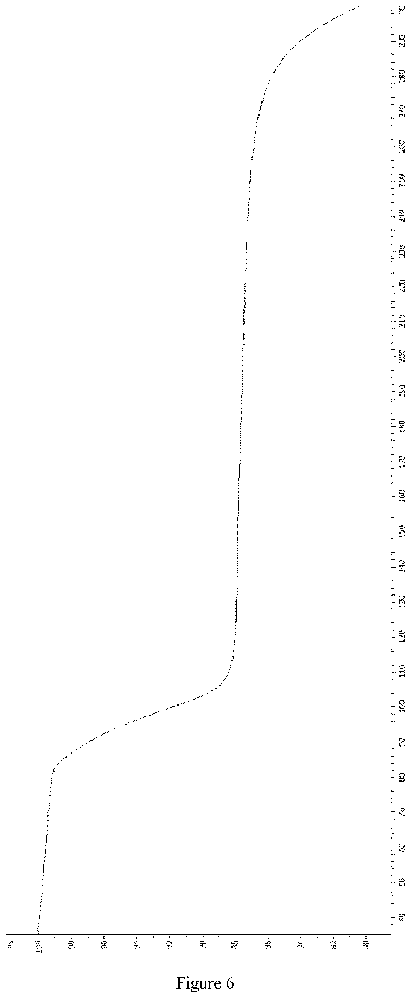

Without limitation, the crystal form B of the present disclosure has a TGA pattern as shown in Figure 6.

Combining the data of DSC and TGA, it is shown that the crystal form B of the present disclosure is an isopropanol solvate.

Without limitation, the crystal form B of the present disclosure has a microscope photograph as shown in Figure 8, and the crystal form B has a columnar crystal habit, a smooth crystal habit surface, and good fluidity.

Another object of the present disclosure is to provide a method for preparing the crystal form B, comprising the following steps:
(1) mixing a crude product of compound of formula (I) with isopropanol;
(2) stirring and crystallizing for 1-24 h;
(3) filtering to obtain the crystal form B.

Preferably, wherein, in the above step (1), a mass volume ratio of the crude product to isopropanol is 1:5-30, and its unit is g/ml; and a temperature for the mixing is 25-80°C. Preferably, a temperature for stirring and crystallizing in step (2) is 5-25°C.

The present disclosure further relates to a pharmaceutical composition containing the crystal form A and/or the crystal form B of compound of formula (I), wherein the pharmaceutical composition comprises a therapeutically effective amount of the crystal form A and/or the crystal form B of compound of formula (I), and one or more pharmaceutically acceptable carrier(s).

The present disclosure also further to use of the crystal form A, the crystal form B of compound of formula (I) or their pharmaceutical composition in the preparation of a medicament for treating the new coronavirus.

The two new crystal forms of crystal form A and crystal form B prepared by the preparation methods of the present disclosure have good physical and chemical stability, regular crystal habits, good particle size uniformity, and good fluidity, which well solves the shortcomings in the existing patented technologies such as cumbersome crystallization process, small particle size, uneven particles and so on, and has excellent properties in terms of processing adaptability. Moreover, the preparation methods of the new crystal forms can effectively remove impurities that are difficult to remove, obtain samples with less impurities and high purity, which can also be used as an intermediate to make the crystallization process of the finished product simpler. The crystallization process of the present disclosure is simple, easy to operate, has small pollution, and has the advantage of being able to achieve industrial production.

### BRIEF DESCRIPTION OF THE DRAWINGS

Figure 1 is an X-ray powder diffraction pattern of the crystal form A obtained in Example 1.
Figure 2 is a DSC pattern of the crystal form A obtained in Example 1.
Figure 3 is a TGA pattern of the crystal form A obtained in Example 1.
Figure 4 is an X-ray powder diffraction pattern of the crystal form B obtained in Example 5.
Figure 5 is a DSC pattern of the crystal form B obtained in Example 5.
Figure 6 is a TGA pattern of the crystal form B obtained in Example 5.
Figure 7 is a microscope photograph of the crystal form A obtained in Example 1.
Figure 8 is a microscope photograph of the crystal form B obtained in Example 5.
Figure 9 is a microscope photograph of the crystal form 1 obtained in the Preparation Example.
Figure 10 is a microscope photograph of the crystal form 2 obtained in the Preparation Example.
Figure 11 is a microscope photograph of the crystal form 4 obtained in the Preparation Example.
Figure 12 is a microscope photograph of the crystal form 5 obtained in the Preparation Example.

### DETAILED DESCRIPTION

The following examples are intended to further explain the present disclosure, and do not constitute a limitation on the scope of the present disclosure.

The raw material for preparing crystal form A or B used in the methods of the present disclosure may use the crude product of compound of formula (I), and the crude product of compound of formula (I) may be prepared by vacuum concentrating a eluent according to step 8 on page 129, line 27 of Example 13 disclosed in patent WO2021250648A1, including but not limited to the above sources.

The solvent used in the present disclosure is not particularly limited, and commercially available conventional solvents may be used.

Unless otherwise specified, the "stirring" described in the methods of the present disclosure may adopt conventional methods in the art, for example, the method for stirring includes magnetic stirring, mechanical stirring, and the stirring speed is 150-300 rpm/min.

The X-ray powder diffraction instrument and test conditions involved in the present disclosure were as follows: X-ray diffraction instrument model MiniFlex600Cu target; operation method: scanning speed 20°/min, scanning step width 0.02°.

The DSC test conditions involved in the present disclosure were as follows: DSC detector model: NETZSCH DSC 214 Polyma; operation method: heating rate 10°C/min, temperature range: 30-250°C.

The TGA test conditions involved in the present disclosure were as follows: TGA detector model: METTLER TOLEDO TGA2; operation method: heating rate 10°C/min, temperature range: 35-300°C.

The OLYMPUS microscope involved in the present disclosure had a model of CX31 (10×10).

The liquid phase test conditions involved in the present disclosure were as follows: chromatographic column: Agilent Zorbax SB-18, 150×4.6 mm, 5 µm; mobile phase A: 0.1% phosphoric acid aqueous solution; mobile phase B: acetonitrile; detection wavelength: 205 nm; flow rate: 1.0 ml/min; injection volume: 5 µl; column temperature: 80°C.

### Elution gradient:

| Time(min) | A(%) | B(%) |
|---|---|---|
| 0 | 75 | 25 |
| 10 | 60 | 40 |
| 20 | 55 | 45 |
| 30 | 20 | 80 |
| 30.1 | 75 | 25 |
| 35 | 75 | 25 |

It should be emphasized that the meaning or intended protection scope of the numerical values or numerical endpoints involved in the technical solution of the present disclosure is not limited to the numbers themselves. Those skilled in the art can understand that the numerical values or numerical endpoints include those allowable error ranges that have been widely accepted in the art, such as experimental errors, measurement errors, statistical errors, random errors, etc., and these error ranges are all included in the scope of the present dislcosure.

### Example 1

1 g of the crude product was added to 0.3 ml of methanol and 10 ml of isobutyl acetate, and evenly mixed, the mixture was heated to 40°C, slowly cooled to 25°C, stirred and crystallized for 2 h, filtered, and 0.87 g of crystals were obtained with a purity of 99.86%, and confirmed as crystal form A by X-ray powder diffraction pattern (XRD).

The patterns of X-ray powder diffraction, DSC and TGA of the crystal form are shown in Figures 1-3, respectively, and the microscope photograph is shown in Figure 7. It is named as crystal form A in the present disclosure.

### Example 2

1 g of the crude product was added to 0.1 ml of ethanol and 10 ml of isobutyl acetate, and evenly mixed, the mixture was heated to 80°C, slowly cooled to 15°C, stirred and crystallized for 1 h, filtered, and 0.89 g of crystals were obtained with a purity of 99.78%, and confirmed as crystal form A by X-ray powder diffraction pattern (XRD).

### Example 3

1 g of the crude product was added to 0.2 ml of methanol and 4.8 ml of isobutyl acetate, and evenly mixed, the mixture was dissolved at 25°C, slowly cooled to 5°C, stirred and crystallized for 24 h, filtered, and 0.85 g of crystals were obtained with a purity of 99.91%, and confirmed as crystal form A by X-ray powder diffraction pattern (XRD).

### Example 4

1 g of the crude product was added to 5 ml of methanol and 35 ml of isobutyl acetate, and evenly mixed, the mixture was dissolved at 25°C, slowly cooled to 5°C, stirred and crystallized for 2 h, filtered, and 0.80 g of crystals were obtained with a purity of 100%, and confirmed as crystal form A by X-ray powder diffraction pattern (XRD).

### Example 5

1 g of the crude product was added to 5 ml of isopropanol and evenly mixed, the mixture was heated to 80°C, slowly cooled to 25°C, stirred and crystallized for 3 h, filtered, and 0.87 g of crystals were obtained with a purity of 99.57%, and confirmed as crystal form B by X-ray powder diffraction pattern (XRD).

The patterns of X-ray powder diffraction, DSC and TGA of this crystal form are shown in Figures 4-6, respectively, and the microscope photograph is shown in Figure 8. It is named as crystal form B in the present disclosure.

### Example 6

1 g of the crude product was added to 20 ml of isopropanol and evenly mixed, the mixture was heated to 35°C, slowly cooled to 15°C, stirred and crystallized for 1 h, filtered, and 0.84 g of crystals were obtained with a purity of 99.73%, and confirmed as crystal form B by X-ray powder diffraction pattern (XRD).

### Example 7

1 g of the crude product was added to 30 ml of isopropanol and evenly mixed, the mixture was dissolved at 25°C, slowly cooled to 5°C, stirred and crystallized for 24 h, filtered, and 0.82g of crystals were obtained with a purity of 99.91%, and confirmed as crystal form B by X-ray powder diffraction pattern (XRD).

### Preparation Example:

Preparation of crystal form 1: crystal form 1 was prepared according to step 2 on page 135 of patent WO2021250648A.

Preparation of crystal form 2: crystal form 2 was prepared with ethyl acetate and methyl tert-butyl ether according to step 8 on page 129, line 28 of Example 13 of patent WO2021250648A.

Preparation of crystal form 4: crystal form 4 was prepared according to steps 1-2 on pages 151-152 of patent WO2021250648A.

Preparation of crystal form 5: crystal form 5 was prepared by concentration with dichloromethane according to Example 96 on page 257 of patent WO2021250648A.

### Comparative Example 1

The samples of crystal form A obtained in Example 1, the crystal form B obtained in Example 5 of the present disclosure, and the crystal forms 1, 2, 4, and 5 obtained in Preparation Example, and crude products were subjected to HPLC detection. The results are shown in Table 1.

**Table 1**

| Crystal form | HPLC results | | | | |
|---|---|---|---|---|---|
| | RRT=0.96 | RRT=1 | RRT=1.04 | Compared with the crude product, the changes of impurities at RRT=0.96 | Compared with the crude product, the changes of impurities at RRT=1.04 |
| Crude product | 1.89 | 91.21 | 1.17 | | |
| Crystal form A | | 99.86 | 0.08 | -1.89 | -1.09 |
| Crystal form B | 0.09 | 99.57 | 0.11 | -1.80 | -1.06 |
| Crystal form 1 | 1.36 | 95.34 | 0.79 | -0.53 | -0.38 |
| Crystal form 2 | 1.68 | 93.83 | 0.87 | -0.21 | -0.30 |
| Crystal form 4 | 1.33 | 93.31 | 0.79 | -0.56 | -0.38 |
| Crystal form 5 | 1.88 | 91.23 | 1.19 | -0.01 | 0.02 |

As can be seen from Table 1, the preparation methods of crystal form A and crystal form B are better than those of crystal form 1, 2, 4, and 5 in removing difficult-to-remove impurities.

### Comparative Example 2

The samples of crystal form A obtained in Example 1, the crystal form B obtained in Example 5 of the present disclosure, and the crystal forms 1, 2, 4, and 5 obtained in Preparation Example were placed at 60°C for 5 days and 10 days, respectively, and subjected to HPLC and XRD detection, respectively. The results are shown in Table 2.

**Table 2**

| Compounds | 0 Day | | 5 Days | | 10 Days | | Changes of HPLC purity over 10 days |
|---|---|---|---|---|---|---|---|
| | HPLC purity (%) | XRD results | HPLC purity (%) | XRD results | HPLC purity (%) | XRD results | |
| Crystal form A | 99.86 | Crystal form A | 99.86 | Crystal form A | 99.85 | Crystal form A | -0.01 |
| Crystal form B | 99.57 | Crystal form B | 99.56 | Crystal form B | 99.54 | Crystal form B | -0.03 |
| Crystal form 1 | 95.34 | Crystal form 1 | 95.34 | Crystal form I | 95.31 | Crystal form I | -0.03 |
| Crystal form 2 | 93.83 | Crystal form 2 | 93.71 | Mixed crystal | 93.52 | Mixed crystal | -0.31 |
| Crystal form 4 | 93.31 | Crystal form 4 | 93.26 | Crystal form 4 | 93.14 | Crystal form 4 | -0.17 |
| Crystal form 5 | 91.23 | Crystal form 5 | 91.05 | Mixed crystal | 90.79 | Mixed crystal | -0.44 |

As can be seen from Table 2, in the case where crystal form A, crystal form B and crystal form I were placed at 60°C for 10 days, the crystal forms and liquid HPLC data were more stable than crystal form 2, crystal form 4 and crystal form 5.

### Comparative Example 3

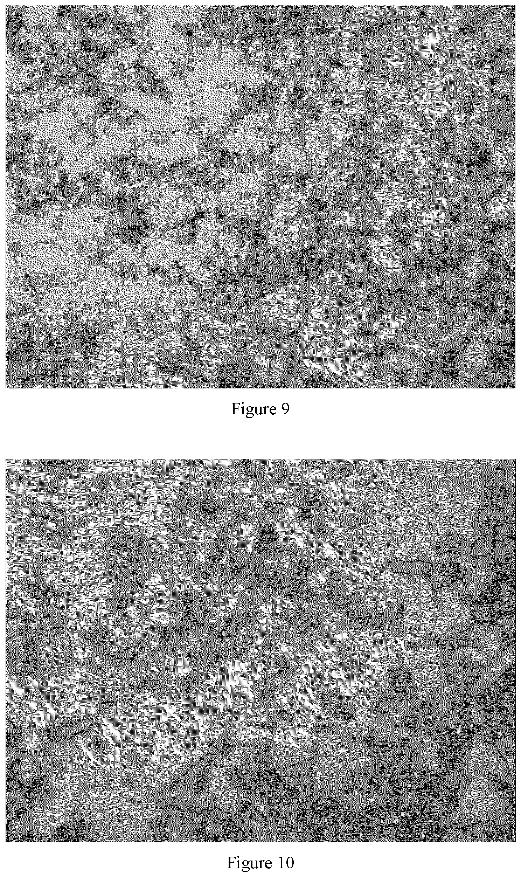

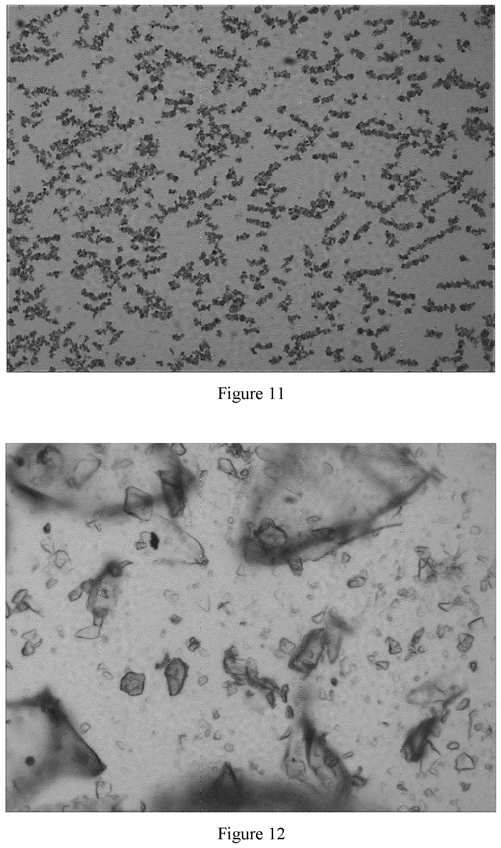

Microscope photographs of the samples of the crystal form A obtained in Example 1, the crystal form B obtained in Example 5 of the present disclosure, and the crystal forms 1, 2, 4, and 5 obtained in Preparation Example are shown in Figures 7-12, respectively.

The crystal form A has uniform crystal particle size, is quasi-spherical, small in specific surface area, small in friction between crystals, and good in particle fluidity. The crystal form B has uniform particle size, is blocky, small in aspect ratio, smooth in crystal surface, and good in particle fluidity. Crystal form 1 is needle-shaped, with a large aspect ratio and poor fluidity. Crystal form 2 is conical, with a large aspect ratio and poor fluidity. Crystal form 4 has uniform particle size, but the particle size is small, which is easy to agglomerate, difficult to filter, resulting in high residual mother liquor, and difficulty in drying. Crystal form 5 does not have uniform particle size, with poor fluidity, and particles are brittle, which is not conducive to subsequent processing. Therefore, crystal forms A and B have better crystal habits, uniform particle sizes, which is easy to store and transport, as well as subsequent processing.

## Claims

1. A crystal form A of compound of formula (I), wherein, X-ray powder diffraction pattern of the crystal form A has characteristic peaks at the following diffraction angles 2θ: 6.2±0.2°, 6.9±0.2°, 7.9±0.2°, 10.5±0.2°, 11.5±0.2°, 11.9±0.2°, 12.5±0.2°, 13.9±0.2°, 14.3±0.2°, 15.8±0.2°, 18.7±0.2°, 19.4±0.2°, 20.7±0.2° and 23.3±0.2°,

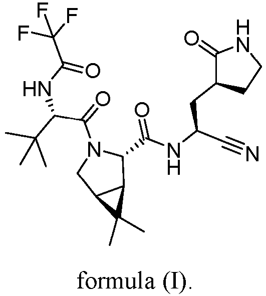

2. The crystal form A according to claim 1, wherein, X-ray powder diffraction pattern of the crystal form A is basically as shown in Figure 1.

3. A method for preparing the crystal form A of any one of claims 1 to 2, wherein, the method comprises the following steps:
(1) mixing a crude product of compound of formula (I) with a mixed solvent of alcohol and isobutyl acetate;
(2) stirring and crystallizing for 1-24 h;
(3) filtering to obtain the crystal form A.

4. The method according to claim 3, wherein, in the step (1), a mass volume ratio of the crude product of compound of formula (I) to the mixed solvent is 1:5-40, and its unit is g/ml; a volume ratio (ml/ml) of the alcohol to isobutyl acetate is 1:7-1:100; the alcohol is C1-C2 alcohol; and a temperature for the mixing is 25-80°C.

5. The method according to claim 3, wherein, a temperature for stirring and crystallizing in the step (2) is 5-25°C.

6. A crystal form B of compound of formula (I), wherein, X-ray powder diffraction pattern of the crystal form B has characteristic peaks at the following diffraction angles 2θ: 6.7±0.2°, 7.4±0.2°, 9.3±0.2°, 10.0±0.2°, 10.9±0.2°, 11.2±0.2°, 15.8±0.2°, 16.4±0.2°, 17.4±0.2°, 18.4±0.2°, 19.4±0.2°, 20.3±0.2° and 20.8±0.2°,

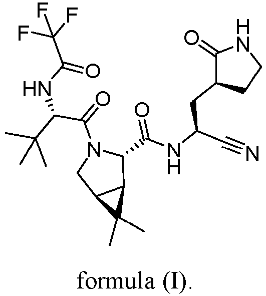

7. The crystal form B according to claim 6, wherein, X-ray powder diffraction pattern of the crystal form B is basically as shown in Figure 2.

8. A method for preparing the crystal form B of any one of claims 6 to 7, wherein, the method comprises the following steps:
(1) mixing a crude product of compound of formula (I) with isopropanol;
(2) stirring and crystallizing for 1-24 h;
(3) filtering to obtain the crystal form B.

9. The method according to claim 8, wherein, in the step (1), a mass volume ratio of the crude product to isopropanol is 1:5-30, and its unit is g/ml; and a temperature for the mixing is 25-80°C.

10. The method according to claim 8, wherein, a temperature for stirring and crystallizing in the step (2) is 5-25°C.

11. A pharmaceutical composition containing the crystal form A of compound of formula (I) of any one of claims 1 to 2 and/or the crystal form B of compound of formula (I) of any one of claims 6 to 7.

12. Use of the crystal form A of compound of formula (I) of any one of claims 1 to 2, the crystal form B of compound of formula (I) of any one of claims 6 to 7 or the pharmaceutical composition of claim 11 in the preparation of a medicament for treating the new coronavirus.
